# EUROPEAN PATENT APPLICATION

(11) **EP 4 851 631 A1**
(43) Date of publication of application: **09.12.2026**
(21) Application number: 25180704.6
(22) Date of filing: 04.06.2025
(51) Int. Cl.: G01N 33/574

(54) **USE OF BMMF1 REP PROTEIN AS DIAGNOSTIC MARKER FOR LIVER CANCER AND LIVER METASTASIS**

(71) Applicant: Deutsches Krebsforschungszentrum Stiftung des öffentlichen Rechts, 69120 Heidelberg (DE)
(72) Inventor: BUND, Timo, 69120 Heidelberg (DE); SIQIN, Sumen, 69120 Heidelberg (DE); RAHBARI, Mohammad, 69120 Heidelberg (DE); DE VILLIERS, Ethel-Michele, 69120 Heidelberg (DE); ERNST, Claudia, 69120 Heidelberg (DE); TESSMER, Claudia, 69120 Heidelberg (DE)
(74) Representative: Schüssler, Andrea

(57) **Abstract**

The present invention relates to the use of BMMF Rep-protein together with CD68 as a diagnostic marker for liver cancer and liver metastasis.

## Description

The invention relates to the use of a DNA-replication-associated (Rep) protein as a diagnostic marker for liver cancer and liver metastasis.

### Background of the Invention

The World Health Organization (WHO) estimates in 2022 that over 15% of different cancers are caused directly or indirectly by infectious agents. Great efforts are undertaken to understand the role of the presence of viruses and other microorganisms as well as environmental or lifestyle factors in cancer development. Hepatocellular carcinoma (HCC) is the most common type of primary liver cancer, representing 85-90% of all cases, and remains the fourth leading cause of cancer-related deaths worldwide. Moreover, the liver is the most frequently affected site for distant metastasis of extrahepatic cancers. Liver metastases are by far the most common liver cancer subtype in the non-cirrhotic liver. In the case of colorectal cancer (CRC), it is estimated that around 50% of the patients develop liver metastasis during the course of the disease (6). Predominant risk factors for the development of primary liver cancer are represented by chronic viral infections with hepatitis B (HBV) or hepatitis C (HCV) virus, or metabolic diseases such as alcoholic liver disease (ALD) or metabolic dysfunction-associated steatotic liver disease (MASLD). Due to the implementation of vaccination programs against HBV infections and the development of effective treatments for chronic HCV infection, presently, metabolic risk factors, which are associated with lifestyle factors such as increased intake of alcohol or high-caloric foods, emerged as the predominant aetiology for liver cancer. The liver is indirectly exposed to environmental factors due to its role in filtering blood that absorbs nutrients, toxins, and pathogens from the digestive tract. This exposure makes it vulnerable to various external substances, including dietary and chemical compounds.

Once a liver tumor or liver metastasis has been identified, chances of healing under present treatment modalities, are limited. Therefore methods to improve early diagnosis are of great importance and diagnostic markers which will facilitate early detection and further insights into the pathogenesis of liver cancer and metastasis are needed

### Description of the Present Invention

The inventors found that the uptake of BMMF (Bovine Meat and Milk Factor) agents within the first months of life either by substitution of breast-feeding during weaning by cow milk products or by the uptake of dairy or beef products, in general, leads to the early infection of newborns with BMMF antigens. The early exposure is thereby hypothesized to induce an immunological state in which the individuals will not clear or neutralize BMMF which is taken up via the nutrition in the following part of life as efficiently as individuals which profited from a longer breastfeeding period. Within the next years or decades - depending on the immune system of the host - additional BMMF antigens accumulate within the stroma of liver tissue. This accumulation may be triggered also by the uptake of specific molecules that represent receptors for BMMFs. These molecules are also taken up by consumption of cow products and are incorporated into lectin receptors on the surface of the host cells. When a certain level of antigen is reached by continuous uptake of BMMFs in combination with focal spreading of infection, the host immune response induces a state of chronic and local inflammation producing a stable increase of diffusing, reactive oxygen species (ROS) and cyclooxygenase-2 (Cox-2) which dramatically increases the probability of deregulated cell proliferation with concomitant fixation of random mutations in surrounding cells induced by ROS and oxidative DNA damage (8, 22). Especially, cells with intrinsically high replicative activity might represent targets enriching random DNA mutations enabling stochastic manifestation of mutations as a basic requirement for tumorigenesis and development of liver cancer. Thus, BMMFs represent a specific and local trigger for induction of chronic inflammation within the tissue stroma leading to an increase of ROS which induces proliferation and mutation in surrounding replicative cells eventually leading to the formation of hyperplasia as precursors for cancer.

For the isolation of the first BMMFs, bovine sera were subjected to density gradient centrifugation. Single fractions of these gradients were analysed by DNA extraction, restriction digestion, cloning of the DNA concatemers/fragments and sequencing. Comparison to all databanks revealed a DNA sequence homology to the transmissible spongiform encephalopathy (TSE)-associated isolates Sphinx 1.76 (1,758 bp; accession no. HQ444404) and Sphinx 2.36 (2,360 bp; acc no. HQ444405, (14)). Full-length plasmid-like, episomal DNA genomes of Bovine Meat and Milk Factors (BMMF) were obtained using abutting primers and PCR amplification on the partial DNA fragments. The main open reading frame of all isolates was identified *in silico* as a putative replication gene (Rep) and revealed a remarkable degree of similarity to specific plasmids of *Acinetobacter baumannii* and *Psychrobacter species.* More than 130 BMMF DNA genomes were subsequently isolated from milk and other dairy products, as well as 3 isolates from a brain biopsy and serum from multiple sclerosis patients. These isolates were grouped as BMMF1, BMMF2 and BMMF4 based on DNA sequence homology (1, 2, 3, 7, 15). Detailed analyses of their genomes revealed characteristics of both viruses and bacterial plasmids needed for replication, transcription and translation, frequently all present in a single genome, thereby constituting a novel class of pathogens (15).

Two BMMF1 isolates from a brain biopsy of a MS patient were MSBI1.176 (MSBI, multiple sclerosis brain isolate) (1,766 bp) and MSBI2.176 (1,766 bp) which are designated as "MSBI1 genome" and "MSBI2 genome", respectively. MSBI1.176 shares 98% nucleotide similarity to the sequence of Sphinx 1.76. The sequences of the isolates MSBI1.176 and MSBI2.176 have been deposited in the EMBL Databank under accession numbers LK931491 (MSBI1.176) and LK931492 (MSBI2.176) and have been aligned and described in WO 2016/005054 A2.

Further isolates were obtained from cow milk, dairy products and bovine sera. These Cow milk isolates (CMI) were CMI1.252, CMI2.214, CMI3.168, CMI4.158, HCBI3.108, HCBI4.296, HCBI6.252, C1MI.3M.1 and C1MI.9M.1 which are designated as "CMI1 genome", "CMI2 genome", "CMI3 genome", "CMI4 genome", "HCBI3 genome", "HCBI4 genome", "HCBI6 genome", "C1MI.3M.1 genome", "C1MI.9M.1 genome", respectively. The sequences of the isolates have been deposited in the EMBL Databank under accession numbers LK931487 (CMI1.252), LK931488 (CMI2.214), LK931489 (CMI3.168), LK931490 (CMI4.158), LK931495 (HCBI3.108), LK931496 (HCBI4.296), LK931493 (HCBI6.252), LR215499 (C1MI.3M.1), LR215496 (C1MI.9M.1) and have been aligned and described in part in WO 2016/005054 A2. The CMI5 genomes are described in (7).

Furthermore, epidemiological studies indicate a correlation between high consumption of bovine meat and milk products and regional incidence of certain cancers, most notably breast and colorectal cancers (CRC) (10,11). Importantly, a link of red meat consumption and liver cancer incidence has also been demonstrated in epidemiological studies (12,13). Bovine meat and milk factors (BMMFs), a group of >140 different plasmid-like DNA molecules was detected in bovine milk and serum, and different cancers as well as from other sources (14-20). BMMFs are bioactive, can replicate, transcribe and translate BMMF-encoded genes in human cells (21). All BMMFs encode a conserved replication initiator protein (Rep). Using monoclonal anti-Rep antibodies and immunohistochemistry (IHC), high Rep expression was found in the tumor-adjacent mucosa of 99% of CRC patients, which was associated with the presence of CD68⁺CD163⁺ M2-like macrophages. Moreover, BMMF expression was increased in CRC patients, and even tumor-preceding pro-tumorigenic low- and high-grade dysplasia, when compared to age-matched healthy individuals, supporting an (early) causal contribution to the development and progression of CRC (22,23). This evidence supported a proposed model that BMMFs are drivers or indirect carcinogens for CRC upon induction of chronic inflammation and random DNA mutation in exposed tissues via indirect carcinogenesis and does not necessarily depend on the persistence of the viral DNA in cancer cells (24,25). In addition, BMMFs were detected in the peritumoral tissue and blood of lung and pancreatic cancer patients (26).

The role of BMMFs in the pathophysiology of liver cancer is elusive. Therefore, the inventors determined whether BMMFs are present in the liver of several patients with HCC and colorectal liver metastasis (CRLM) and investigated whether BMMFs are linked to chronic inflammation and chronic inflammation-associated carcinogenesis in the liver. The inventors observed the expression of the Rep protein in all integrated HCC and CRLM cases by immunohistochemistry (IHC). In addition, an increased number of Rep⁺CD68⁺ macrophages was present in HCC and CRLM compared to healthy liver tissue specimens. These observations indicate a potential pathophysiological role of BMMFs in chronic inflammation-related liver malignancies.

The present inventors demonstrated previously that both CMI genomes and MSBI genomes replicate and show a significant production of transcribed RNA in vitro. Antibodies against the MSBI1.176 Rep show Rep protein expression in the liver cancer tissue as such and the peripheral tissue around the liver cancer tissue. The present inventors have found that the encoded Rep proteins of BMMF1 infectious agents (MSBI1 Rep, MSBI2 Rep, CMI1 Rep, CMI2 Rep, CMI3 Rep, CMI4 Rep, CMI5 Rep, HCBI4 Rep, HCBI6 Rep, C1MI.3M.1 Rep, C1MI.9M.1 Rep) represent a diagnostic marker for liver cancer and metastasis.

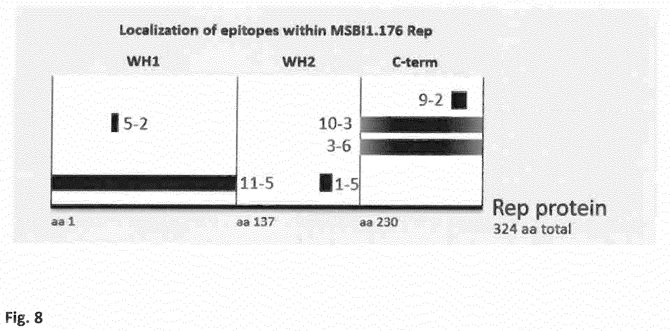

The inventors have raised monoclonal antibodies against BMMF1 Rep protein using full-length MSBI1.176 Rep or consensus peptides derived from two conserved regions of the MSBI1.176 Rep amino acid sequence. In particular embodiments the anti-Rep antibodies bind to epitopes of MSBI1.176 Rep protein that are exemplified in Fig. 8. Particular preferred antibodies bind to epitopes within an amino acid sequence selected from the group consisting of amino acids from 1 to 136, from 137 to 229 and from 230 to 324 of SEQ ID NO:1. For example, the antibody binds to an epitope comprised by SEQ ID NO:2 or SEQ ID NO:3.

### Brief description of the Figures:

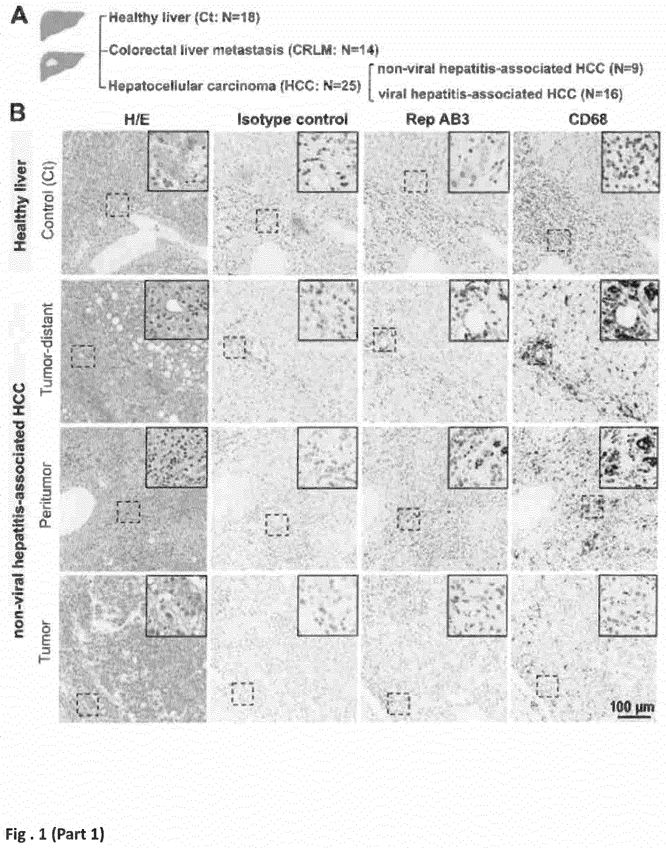

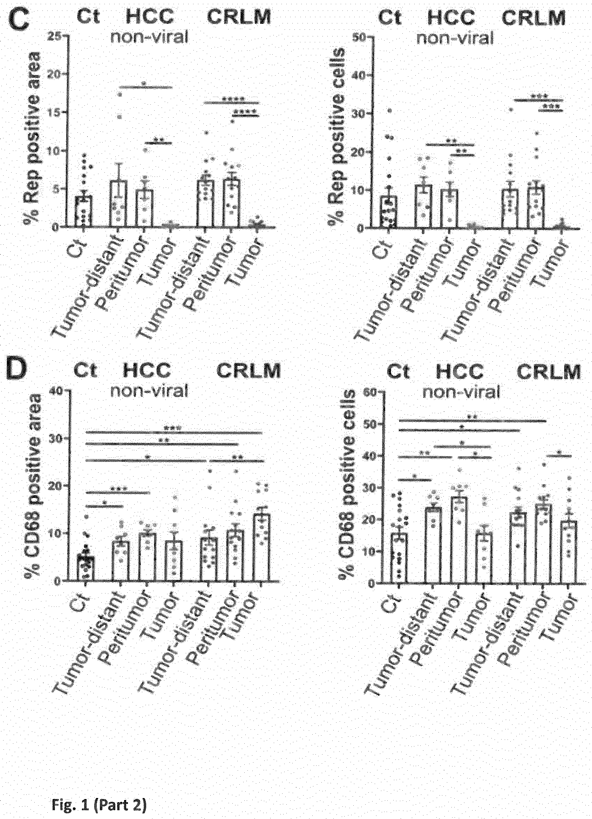

- Fig. 1: **Immunohistochemical (IHC) detection of the BMMF Rep protein in the human liver. (A)** Overview of tissue cohorts used for immunohistochemistry. **(B)** H/E staining and IHC DAB detection for isotype control, anti-Rep and anti-CD68 antibodies on consecutive FFPE tissue sections of healthy liver and tumor-distant, peritumor, and tumor tissue of patients with non-viral hepatitis-associated HCC and CRLM (scale bar: 100 µm, magnification of enlargements: 2.5 x). **(C)** Percentage of Rep positive tissue area per total analyzed area (left panel) and % positive cells of all analyzed cells (right panel). **(D)** Percentage of positive area (left panel) and cells (right panel) for CD68. Data represented by mean+/- SEM. Each dot indicates one individual sample (Ct N=18; non-viral HCC: tumor-distant N=8, peritumor N=7, tumor N=9; CRLM: tumor-distant N=14, peritumor N=14, tumor N=12). *P ≤ 0.05, ** P ≤ 0.01, *** P ≤ 0.001, **** P ≤ 0.0001. H/E: Hematoxylin and eosin staining.

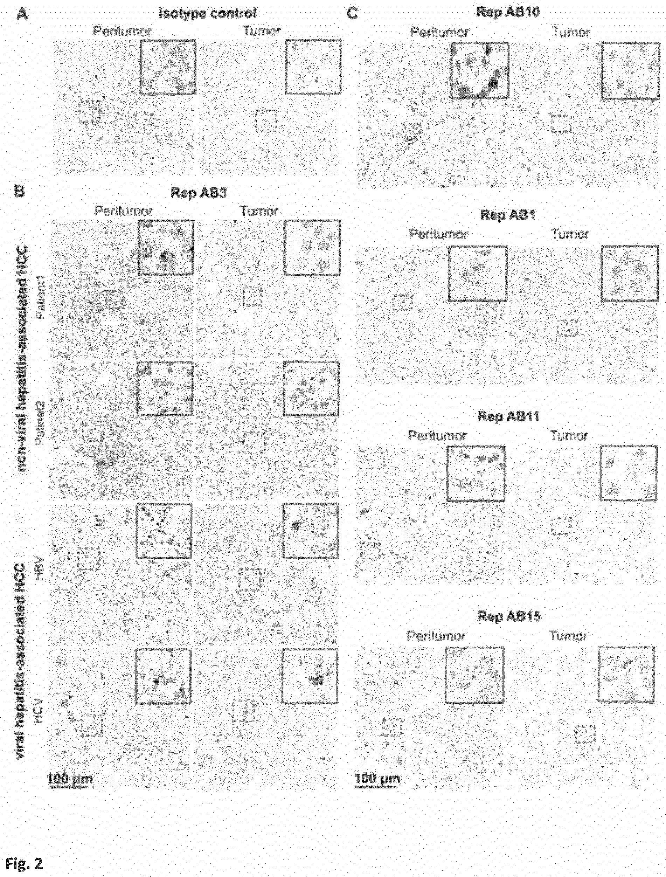

- Fig. 2: **Anti-BMMF Rep IHC detection in peritumor and tumor tissues of viral and non-viral hepatitis-associated HCC. (A-B)** IHC DAB detection with isotype control and anti-Rep AB3 antibodies of tissue specimens from 2 non-viral and 2 viral hepatitis-associated HCC patients. **(C)** IHC detection with different anti-Rep antibodies (AB10, AB1, AB11 and AB15) in representative (paired) peritumor and tumor tissue of HCC patient1 (scale bar: 100 µm, magnification of enlargements: 2.5x).

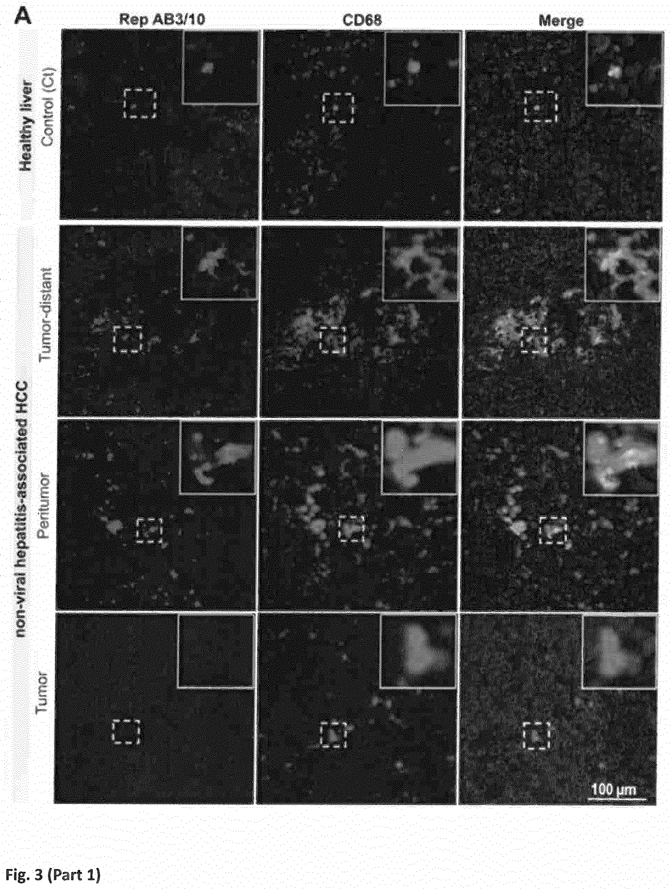

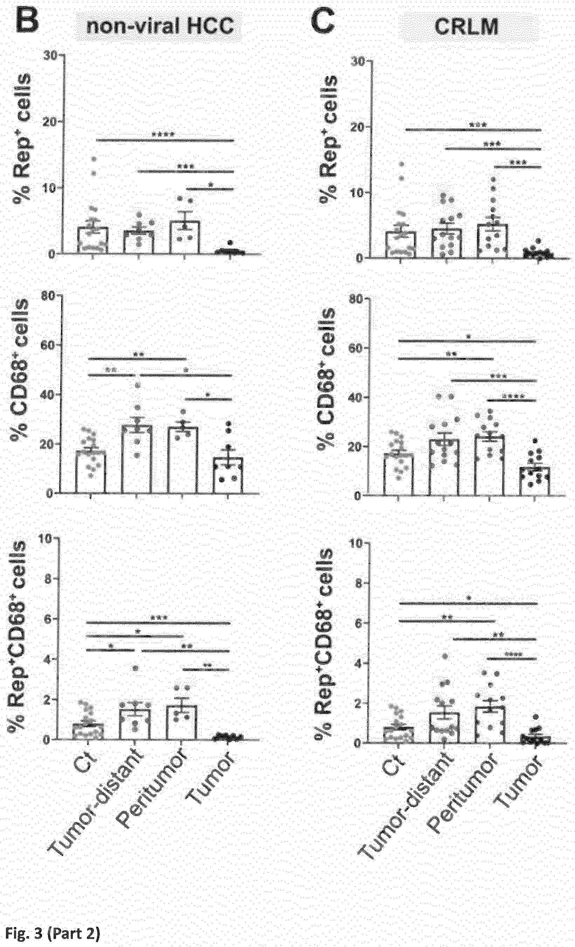

- Fig. 3: **Co-immunofluorescence (IF) detection of Rep⁺ and CD68⁺ cells in the portal triad (PT) region. (A)** Representative images of IF detection for DAPI (blue), anti-Rep (AB3 and 10, green) and anti-CD68 (red) in healthy liver and tissues of non-viral hepatitis-associated HCC (scale bar: 100 µm, magnification of enlargements: 2.5x). **(B-C)** Quantification of % Rep⁺, CD68⁺ and Rep⁺CD68⁺ double positive cells in healthy livers and tumor-distant, peritumor and tumor tissues of non-viral hepatitis-associated HCC and CRLM patients. Data presented by mean+/-SEM. Each dot indicates one individual/sample (Ct N=18; non-viral HCC: tumor-distant N=8, peritumor N=5, tumor N=8; CRLM: tumor-distant N=14, peritumor N=13, tumor N=12). *P ≤ 0.05, ** P ≤ 0.01, *** P ≤ 0.001, **** P ≤ 0.0001.

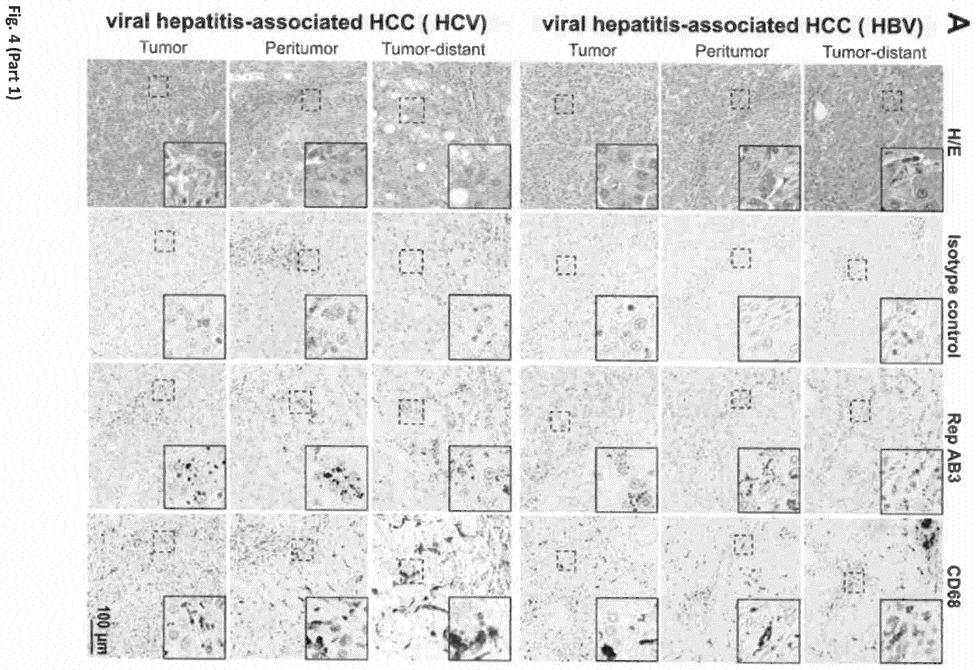

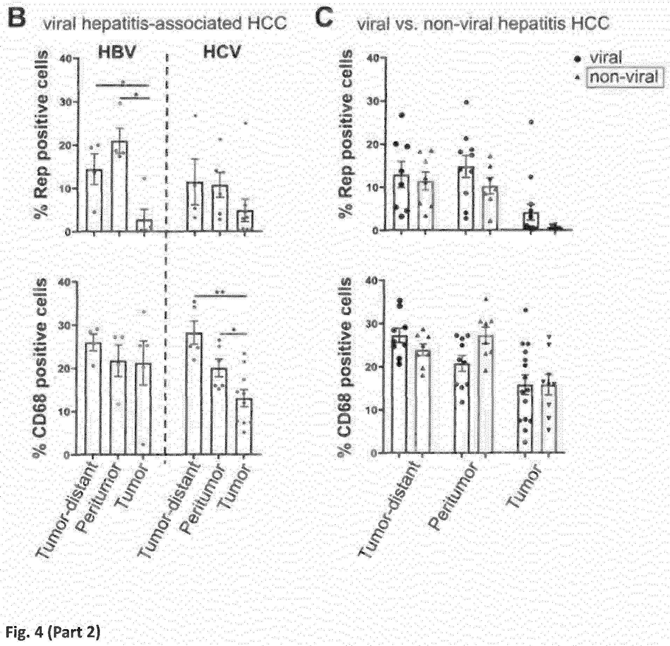

- Fig. 4: **Immunohistochemical detection and quantification of Rep⁺ and CD68⁺ cells in HBV- and HCV-associated HCC. (A)** H/E and IHC detection with isotype control, anti-Rep and anti-CD68 antibodies in the tissues of HBV- and HCV-associated HCC (scale bar: 100 µm, magnification of enlargements: 3x). **(B)** Quantification of Rep⁺ (upper panel) and CD68⁺ (lower panel) cells in HBV- and HCV-associated HCC. **(C)** Quantification of Rep⁺ (upper panel) and CD68⁺ (lower panel) cells in viral and non-viral hepatitis-associated HCC. Data is presented by mean+/-SEM. Each dot indicates one individual/sample (HBV-associated HCC: tumor-distant N=4, peritumor N=4, tumor N=5; HCV-associated HCC: tumor-distant N=5, peritumor N=6, tumor N=10; non-viral HCC: distant N=8, peritumor N=5, tumor N=8). *P ≤ 0.05, ** P ≤ 0.01.

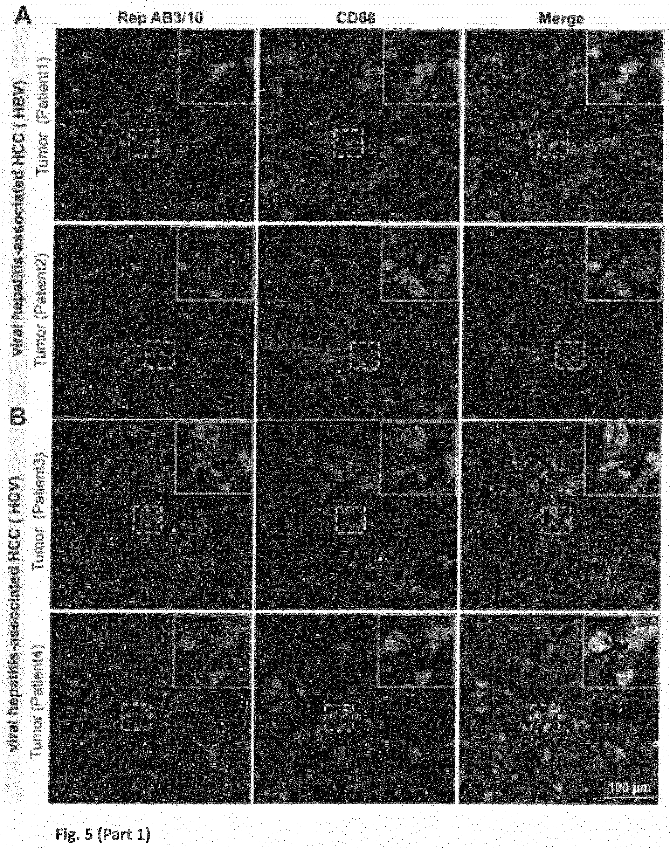

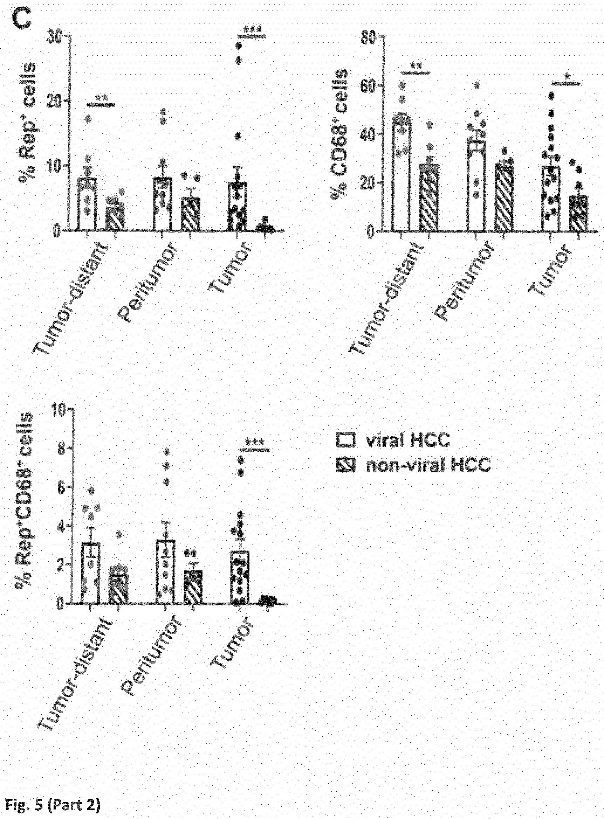

- Fig. 5: **Co-immunofluorescence (IF) detection and comparison of Rep⁺ CD68⁺ cells in viral and non-viral hepatitis-associated HCC. (A-B)** Representative images of IF detection in the tumor of viral hepatitis-associated HCC (scale bar: 100 µm, magnification of enlargements: 2.5x). **(C)** Quantification of IF detection of Rep⁺, CD68⁺ and Rep⁺CD68⁺ positive cells in tumor-distant, peritumor and tumor tissues of viral and non-viral hepatitis-associated HCC (dashed bar) in the portal triad region. Data presented by mean+/- SEM. Each dot indicates one individual/sample (viral-HCC: tumor-distant N=8, peritumor N=10, tumor N=15; non-viral HCC: tumor-distant N=8, peritumor N=5, tumor N=8). *P ≤ 0.05, ** P ≤ 0.01, *** P ≤ 0.001.

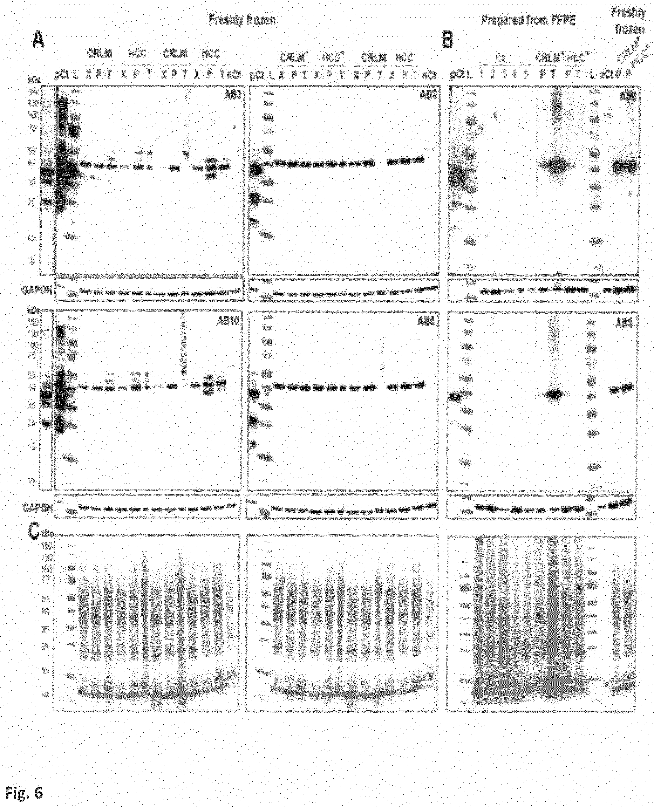

- Fig. 6: **Immunodetection of BMMF Rep protein in liver tissue lysates. (A)** Representative WB images of anti-Rep antibodies AB3/10/2/5 in immunodetections using lysates of freshly frozen liver tissues of CRLM (black) and non-viral hepatitis-associated HCC (blue) showing bands mainly at 45 kDa, additional bands at 38 kDa (reflecting the size of pCt) and higher bands around 55 kD occasionally (25 µg protein lysate per lane). **(B)** Immunodetection of Rep in lysates prepared from FFPE tissues sections of healthy liver (Ct) and the donors of non-viral HCC and CRLM analyzed on the level of freshly frozen tissues from part A (consistently marked with an asterisk*) (25 µg protein lysate per lane). **(C)** Ponceau staining of protein lysates corresponding to panel A and B. (Tissue lysates from X: Tumor-distant, P: Peritumor, T: tumor. Ct: healthy liver controls. L: Protein ladder; pCt: positive control, lysate of HEK293TT cells overexpressing H1MSB.1 Rep; nCt: negative control, lysate of HEK293TT cells overexpressing pcDNA. CRLM: Colorectal liver metastasis, HCC: Hepatocellular carcinoma patients (here refers to non-viral hepatitis-associated HCC).

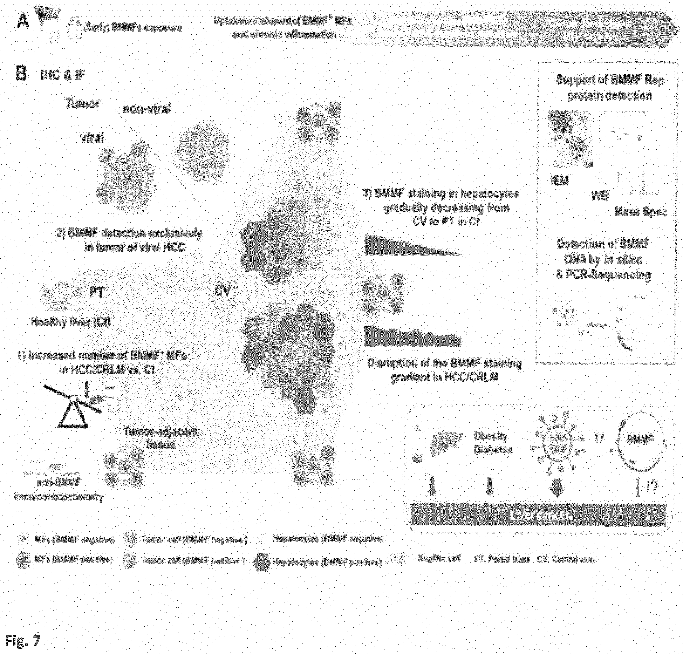

- Fig. 7: **Model of BMMF-driven indirect carcinogenesis and summary of BMMF detection on liver cancer.**
- Fig. 8: **Localization of the antibody epitopes within MSBI1.176 Rep**

The invention provides the teaching that BMMF1 Rep proteins may represent diagnostic markers for an enhanced risk to develop liver cancer, i.e. for the determination of a predisposition for the development of liver cancer. In addition, the invention provides the teaching that Rep proteins may represent diagnostic markers for diagnosing liver cancer or liver metastases, or confirming a diagnosis of liver cancer or liver metastasis.

The term "liver cancer", also known as hepatic cancer, is cancer that starts in the liver or has invaded into the liver. Liver cancer can be primary in which the cancer starts in the liver, or it can be secondary where the cancer spreads from elsewhere in the body to the liver. The secondary form is called "liver metastasis". Primary liver cancer can come from the liver parenchyma as well as other structures within the liver such as the bile duct, blood vessels and immune cells. The most common types are hepatocellular carcinoma (HCC) [which makes up 80% of cases], intrahepatic cholangiocarcinoma (CCA), liver angiosarcoma, liver hemangiosarcoma and hepatoblastoma. HCC is a cancer formed by liver cells, known as hepatocytes, that become malignant. Intrahepatic CCA is an epithelial cancer of the intra-hepatic biliary tree branches. Angiosarcoma and hemangiosarcoma of the liver come from the blood vessel's endothelial layer. Another type of cancer formed by liver cells is hepatoblastoma, which is specifically formed by immature liver cells. Many cancers found in the liver are not true liver cancers but are secondary cancers from other sites in the body that have spread to the liver (known as metastases). Frequently, the site of origin is the gastrointestinal tract, since the liver is close to many of these metabolically active, blood-rich organs near to blood vessels and lymph nodes (such as pancreatic cancer, stomach cancer, colon cancer and carcinoid tumors mainly of the appendix), but also from breast cancer, ovarian cancer, lung cancer, renal cancer, prostate cancer.

The present invention encompasses the systematic testing of presumably healthy liver tissue (tissue from individuals without cancer diagnosis or a specific hint for the disease) to assess the disease risk in the future. This means that the present invention is suitable to determine the predisposition for developing liver cancer by use of the BMMF Rep as an early diagnostic marker.

"Predisposition" means, according to the accepted meaning in medicine, the tendency to a medicinal condition. It is the medicinal susceptibility to a specific disease or a condition of special susceptibility to a disease, usually based on the combined effects of genetic and environmental factors.

"Rep protein" as used herein refers to a DNA-replication-associated protein. The Rep protein comprises DNA binding activity and could be essential for initiation of replication of episomal/viral DNA molecules. In general Rep protein refers to a Rep protein from the group of BMMF1 proteins (15). In particular, the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep),CMI3 genome-encoded Rep protein (CMI3 Rep) or CMI4 genome-encoded Rep protein (CMI4 Rep), HCBI4 genome-encoded Rep protein (HCBI4 Rep), HCBI6-encoded Rep protein (HCBI6 Rep), C1MI.3M.1 genome-encoded Rep protein (C1MI.3M.1 Rep), C1MI.9M.1 genome encoded protein (C1MI.9M.1 Rep). Preferably, the MSBI1 Rep protein is encoded by MSBI1.176 deposited in the EMBL databank under the acc. no. LK931491 and has the amino acid sequence as depicted in SEQ ID NO:1 or the Rep protein is MSBI2 encoded by MSBI2.176 deposited in the EMBL databank under the acc. no. LK931492 and has the amino acid sequence as depicted in SEQ ID NO:8 (2, 3). In another preferred embodiment the CMI1 Rep protein is encoded by CMI1.252 deposited in the EMBL databank under the acc. no. LK931487 and has the amino acid sequence as depicted in SEQ ID NO:10. In another preferred embodiment the CMI2 Rep protein is encoded by CMI2.214 deposited in the EMBL databank under the acc. no. LK931488 and has the amino acid sequence as depicted in SEQ ID NO:11. In another preferred embodiment the CMI3 Rep protein is encoded by CMI3.168 deposited in the EMBL databank under the acc. no. LK931489 and has the amino acid sequence as depicted in SEQ ID NO:12. In another preferred embodiment the CMI4 Rep protein is encoded by CMI4.158 deposited in the EMBL databank under the acc. no. LK931490 and has the amino acid sequence as depicted in SEQ ID NO:16. In another preferred embodiment the HCBI3 is encoded by HCBI3.108 deposited in the EMBL databank under the acc. no. LK931495 and has the amino acid sequence as depicted in SEQ ID NO:17. In another preferred embodiment the HCBI4 is encoded by HCBI4.296 deposited in the EMBL databank under the acc. no. LK931496 and has the amino acid sequence as depicted in SEQ ID NO:18. In another preferred embodiment the C1MI.3M.1 is deposited in the EMBL databank under the acc. no. LR215499 and has the amino acid sequence as depicted in SEQ ID NO:19. In another preferred embodiment the C1MI.9M.1 is deposited in the EMBL databank under the acc. no. LR215496 and has the amino acid sequence as depicted in SEQ ID NO:20.

In a particular preferred embodiment the Rep protein comprises a N-terminal region conserved among BMMF1 genomes consisting essentially of amino acids from 1 to 229 of SEQ ID NO:1 and a C-terminal variable region specific for MSBI1.176 consisting essentially from amino acids 230 to 324 of SEQ ID NO:1. The N-terminal conserved region comprises a putative, first DNA binding domain consisting essentially of amino acids from 1 to 136 of SEQ ID NO: 1 and a second putative DNA binding domain consisting essentially of amino acids from 137 to 229 of SEQ ID NO:1. The C-terminal domain shows little sequence homology with any known protein and consists of amino acids 230 to 324.

"Rep protein" also encompasses fragments and variants of the protein with SEQ ID NO:1 or SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20 which are capable of binding an anti-Rep antibody specific for Rep protein having the amino acid sequence of SEQ ID NO:1. Preferably, such a fragment is an immunogenic fragment of the protein having the amino acid sequence of SEQ ID NO:1, SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20 which encompasses at least one epitope for an anti-Rep protein antibody against the Rep protein of SEQ ID NO:1 and, preferably, comprises at least 7, 8, 9, 10, 15, 20, 25 or 50 contiguous amino acids. In particular embodiments the fragment comprises or consists essentially of a domain of the Rep protein, for example, the N-terminal conserved region, the C-terminal variable region, the first or second DNA binding domain. A variant of the protein with SEQ ID NO:1, SEQ ID NO:8 SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20 comprises one or more amino acid deletions, substitutions or additions compared to the respective sequence and has a homology of at least 90%, 91%, 92%, 93%, 94%, 95%, 96%, 97%, 98% or 99% to the amino acid sequence of SEQ ID NO:1, SEQ ID NO:8, SEQ ID.NO:10, SEQ ID. NO: 11, SEQ ID. NO:12, SEQ ID.NO: 16, SEQ ID.NO:17, SEQ ID NO:18, SEQ ID NO:19 and/or SEQ ID.NO:20, wherein the variant is capable of binding an anti-Rep antibody specific for a Rep protein having the amino acid sequence of SEQ ID NO:1. Included within the definition of variant are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring. The term Rep protein includes fusion proteins with a heterologous amino acid sequence, with a leader sequence or with a Tag-sequence and the like. In certain embodiments of the invention protein tags are genetically grafted onto the Rep protein described above, for example the Rep protein selected from the group consisting of MSBI1, MSBI2, CMI1, CMI2, CMI3, CMI4, HCBI4, HCBI6, C1MI.3M.1 or C1MI.9M.1. In particular at least one protein tag is attached to a polypeptide consisting of an amino acid sequence as depicted in any one of SEQ ID NOs:1-3,8-12,14 and 16-20. Such protein tags may be removable by chemical agents or by enzymatic means. Examples of protein tags are affinity or chromatography tags for purification. For example, the Rep protein may be fused to a Tag-sequence, for example, selected from the group consisting of His₆-Tag (SEQ ID NO:4), T7-Tag (SEQ ID NO:5), FLAG-Tag (SEQ ID NO:6) and Strep-II-Tag (SEQ ID NO:7). a His-Tag (SEQ ID No:4), a T7-Tag (SEQ ID NO:5), FLAG-Tag (SEQ ID NO:6) or Strepll-Tag (SEQ ID NO:7). Further, fluorescence tags such as green fluorescence protein (GFP) or its variants may be attached to a Rep-protein according to the invention.

In a particular preferred embodiment the MSBI1 genome-encoded Rep protein (MSBI1 Rep) is codon-optimized for the production in human cell lines (e.g. HEK-293, HEK293TT, HEK293T, HEK293FT, HaCaT, HeLa, PANC-1, A-549, SiHa, CaSki, HDMEC, L1236, L428, BJAB, MCF7, Colo678, CACO2, DLD-1, HCT116, RKO, THP-1) as well as bovine (e.g. MAC-T) or murine cell lines (e.g. GT1-7) or any other cell line. This is described in detail in PCT/EP2017/075774.

The Rep protein of the invention, including the Rep fragments and Rep variants as defined above, can be prepared by classical chemical synthesis. The synthesis can be carried out in homogeneous solution or in solid phase. The polypeptides according to this invention can also be prepared by means of recombinant DNA techniques.

"Subject" as used herein refers to a mammalian individual or patient, including mice, cattle, for example different bovine species, simians and humans. Preferably, the subject is a human patient.

"Anti-Rep antibody" as used herein refers to an antibody binding at a detectable level to Rep protein which affinity is more strongly to the Rep protein of the invention than to a non-Rep protein. Preferably, the antigen affinity for Rep protein is at least 2-fold, preferably 5- fold or 10-fold, larger than background binding. In particular, the anti-Rep antibody detects the MSBI1 Rep having the amino acid sequence of SEQ ID NO:1. In particular embodiments the antibody detects MSBI1 Rep, MSBI2 Rep, CMI1 Rep, CMI2 Rep, CMI3 Rep, CMI4 Rep, HCBI4, HCBI6, C1MI.3M.1 and/or C1MI.9M.1. In certain embodiments the anti-Rep antibody cross-detects at least two, preferably all, of MSBI1 Rep, MSBI2 Rep, CMI1 Rep, CMI2 Rep, CMI3 Rep, CMI4 Rep, HCBI4, HCBI6, C1MI.3M.1, and/or C1MI.9M.1.

The inventors also tested the antibody level of liver cancer or liver metastasis patients by contacting the Rep protein with a specimen suspected of containing anti-Rep protein antibodies under conditions that permit the Rep protein to bind to any such antibody present in the specimen. Such conditions will typically be physiologic temperature, pH and ionic strength using an excess of Rep protein. The incubation of the Rep protein with the specimen is followed by detection of immune complexes comprised of the antigen. In certain embodiments either the Rep protein is coupled to a signal generating compound, e.g. detectable label, or an additional binding agent, e.g. secondary anti-human antibody, coupled to a signal generating compound is used for detecting the immune complex.

Thus, in a preferred embodiment, the present invention provides a method for diagnosing, or determining a predisposition for, liver cancer or liver metastasis in a human subject, comprising:
- detecting whether a Bovine Meat and Milk Factor Group 1 (BMMF1) DNA-replication associated protein (Rep protein) is present in a sample isolated from a subject (preferably human subject), comprising
- contacting the sample with an anti-BMMF1 Rep antibody and an anti-CD68 antibody;
- detecting binding between BMMF 1 Rep protein and the anti-BMMF1 Rep antibody, wherein detecting the binding between BMMF1 Rep protein and the anti-BMMF1 Rep antibody indicates that BMMF1 Rep is present in the sample,
- detecting binding between CD68-positive cells and the anti-CD68 antibody, wherein detecting the binding between the CD68-positive cells and the anti-CD68 antibody indicates that CD68-positive cells are present in the sample, and
- providing the diagnosis of liver cancer/metastasis or the predisposition for liver cancer to the subject when the presence of both of BMMF1 Rep protein and CD68-positive cells (preferably CD68⁺ macrophages) in the sample is detected.

Rep proteins can be detected and quantified in assays (as antigens) based on anti-Rep antibodies, which serve for detection of the mammalian, e.g. human, antigens suspected in the specimen (or antigens from other sources). Preferably, the presence of Rep protein is tested in samples such as full blood, serum or plasma or tissue lysate. The methods include immobilization of the sample (including the Rep protein) on a matrix followed by incubation of the immobilized Rep protein with anti BMMF antibodies. Finally, the Rep-bound antibodies of the formed immunological complex between Rep protein and antibodies of the specimen are quantified by a detection binding agent coupled to a signal generating compound, e.g. secondary HRP-(horseradish-peroxidase)-coupled detection antibody allowing for HRP-substrate based quantification. This signal generating compound or label is in itself detectable or may be reacted with an additional compound to generate a detectable product.

Design of the immunoassay is subject to a great deal of variation, and many formats are known in the art. Protocols may, for example, use solid supports, or immunoprecipitation. Most assays involve the use of binding agents coupled to signal generating compounds, for example labelled antibody or labelled Rep protein; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labelled and mediated immunoassays, such as ELISA assays.

The immunoassay may be in a heterogeneous or in a homogeneous format, and of a standard or competitive type. Both standard and competitive formats are known in the art. In an additional embodiment, detection of anti-BMMF antibodies in samples (preferably in human blood, serum, plasma) using purified BMMF Rep protein (immobilized on a matrix) in an ELISA-based format and using anti-human secondary HRP-coupled antibody detection systems can be applied.

In an immunoprecipitation or agglutination assay format the reaction between the Rep protein and the anti-Rep antibody forms a network that precipitates from the solution or suspension and forms a visible layer or film of precipitate. If no anti-Rep antibody is present in the specimen, no visible precipitate is formed.

"Sample" as used herein refers to a biological sample encompassing cancerous liver tissue, peripheral tissue surrounding the cancerous tissue and pre-cancerous dysplastic or cirrhotic lesions. The samples encompass tissue samples such as tissue cultures or biopsy specimen. In addition, the term "sample" encompasses blood serum or plasma.

Such methods (ex-vivo or in-vitro) comprise the steps of detecting Rep protein in a sample from a subject by anti-Rep antibodies. In such methods Rep protein is detected in tissue samples by immunohistochemical methods or immunofluoresence microscopy. In such methods anti-Rep antibodies are used for the detection or capturing of the Rep protein in the sample.

The term "antibody", preferably, relates to antibodies which consist essentially of pooled polyclonal antibodies with different epitopic specificities, as well as distinct monoclonal antibody preparations. As used herein, the term "antibody" (Ab) or "monoclonal antibody" (Mab) is meant to include intact immunoglobulin molecules as well as antibody fragments (such as, for example, Fab and F(ab')2 fragments) which are capable of specifically binding to Rep protein. Fab and F(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies useful for the purposes of the present invention include chimeric, single chain, multifunctional (e.g. bispecific) and humanized antibodies or human antibodies.

In certain embodiments the antibody or antigen binding fragment thereof is coupled to a signal generating compound, e.g., carries a detectable label. The antibody or antigen binding fragment thereof can be directly or indirectly detectably labelled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

In certain embodiments BMMF protein detection is possible based on an anti-Rep antibody--coupled contrast reagent (reacting with Rep-positive cells) in low dose-CT.

Anti-Rep antibodies are, preferably, raised (generated) against a Rep protein having the amino acid sequence of SEQ ID NO:1 or a fragment thereof by methods well known to those skilled in the art.

In certain embodiments anti-Rep antibodies are used in the methods of the invention which are capable of binding to several or all kinds of Rep proteins from the group of the BMMF1 proteins. Such antibody binds to an epitope within the conserved N-terminal region of the Rep protein from amino acids 1 to 229 of SEQ ID NO:1. In particular embodiments anti-Rep antibodies of the anti-BMMF1 Rep type are used which bind to an epitope within SEQ ID NO:2 (amino acids 32-49 of SEQ ID NO:1) or SEQ ID NO:3 (amino acids 197-216 of SEQ ID NO:1). The peptide fragments of SEQ ID NO:2 and SEQ ID NO:3 are highly conserved among the Rep proteins from the BMMF1 and appear to be exposed due to their hydrophilic character. Anti-Rep antibodies of this anti-BMMF1 type may be produced by immunization, for example of mice or guinea pig, by peptides consisting essentially of the amino acid sequences as depicted in SEQ ID NOs:2 or 3; or by other immunogenic fragments, preferably comprising at least 8-15 amino acids, derived from the conserved N-terminal Rep protein region from amino acids 1 to 229 of SEQ ID NO:1.

In further embodiments anti-Rep antibodies specific for MSBI1 Rep protein are used. Such antibodies may be produced, for example, by immunization of a mammal such as mice or guinea pig with a full-length Rep protein having the amino acid sequence of SEQ ID NO:1.

Preferably, the methods of the invention use anti-Rep antibodies which are capable of detecting Rep protein up to ranges from picogram to femtogram.

Examples of such groups of anti-Rep antibodies are shown in Table 1:

| **Antibody Group** | **Rep-Protein Localisation** | **Detection** | **Antibody** | **DSMZ deposit** |
|---|---|---|---|---|
| **Group A** | cytoplasm + nuclear membrane (+nucleus) | BMMF1 Reps | AB01 523-1-1 (Ab 1-5; AB1) | DSM ACC3327 |
| | | | Immunisation with the Peptide AB01. | |
| **Group B** | speckles in cytoplasm | BMMF1 Reps | AB02 304-4-1 (Ab 5-2; AB5) | DSM ACC3328 |
| | | | AB02 154-11-5 (Ab 2-4; AB2) | |
| | | | AB02 265-1-7 (Ab 14, AB14) | |
| | | | AB15 AB02 209-13-3 (Ab 15, AB15) | |
| | | | All these antibodies recognize the same epitope since they are derived from the immunisation with the identical peptide (AB02). | |
| **Group C** | cytoplasm + nuclear membrane (+ nucleus) | MSBI1 Rep | MSBI1 381-6-2 (Ab 3-6; AB3) | DSM ACC3329 |
| | | | MSBI1 572-13-19 (Ab 10-3; AB10) | |
| | | | MSBI1 617-1-3 (Ab 11-5; AB11) | |
| **Group D** | speckles in cytoplasm | MSBI1 Rep | D1: MSBI1 961-2-2 (Ab 9-2; AB9) | DSM ACC3331 |
| | | | D2: MSBI1 761-5-1 (Ab 13; AB13) | DSM ACC3330 |

The localization of the antibody epitopes within MSBI1.176 Rep is shown in Fig. 8.

Anti-Rep antibodies of group A have an epitope within the amino acid sequence depicted in SEQ ID NO:3 (aa 198-217 of SEQ ID NO:1) and are capable of detecting MSBI1 Rep and Rep proteins comprising this conserved epitope of the BMMF1 group (e.g. MSBI2, CMI1, CMI2, CMI3, CMI4, HCBI4, HCBI6, C1MI.3M.1, C1MI.9M.1). In immunofluoresence assays such anti-Rep antibodies detect a specific Rep localisation pattern, wherein the main localisation is homogeneously distributed over the cytoplasm and nuclear membrane; and additional weak and homogeneously distributed localisation is seen in the nucleus. An example of such a group A antibody is antibody AB01 523-1-1 (also called antibody 1-5 or AB1; DSM ACC3327) which was employed in the examples as group A antibody.

Anti-Rep antibodies of group B have an epitope within the amino acid sequence depicted in SEQ ID NO:2 (aa 33-50 of SEQ ID NO:1) and are capable of detecting MSBI1 Rep and Rep proteins comprising this conserved epitope of the BMMF1 group (e.g. MSBI2, CMI1, CMI2, CMI3, CMI4, HCBI4, HCBI6, C1MI.3M.1, C1MI.9M.1). In immunofluoresence assays such anti-Rep antibodies detect specifically speckles (cytoplasmatic aggregations) of the Rep protein (often in the periphery of the nuclear membrane). An example of such a group B antibody is the antibody designated as AB02 304-4-1 (also called antibody 5-2 or AB5; DSM ACC3328) which was employed in the examples as group B antibody. Antibody AB2 recognizes the same epitope as AB5 and may be used instead of AB5 or in addition.

Anti-Rep antibodies of group C detect specifically a structural epitope of MSBI1 (SEQ ID NO:1). In immunofluoresence assays such anti-Rep antibodies detect a specific Rep localisation pattern, wherein the main localisation is homogeneously distributed over the cytoplasm and nuclear membrane; and additional weak and homogeneously distributed localisation is seen in the nucleus. An example of such a group C antibody is antibody MSBI1 381-6-2 (also called antibody 3-6 or AB3; DSM ACC3329) which was employed in the Example as group C antibody with an epitope in the sequence of aa 230-324. Another example of an antibody of a group C antibody is antibody MBSI1 572-13-19 (also called antibody 10-3 or AB10) detecting an epitope in the C-terminal domain of MSBI 1 Rep (aa 230-324). Another example of an antibody of a group C antibody is antibody MBSI1 617-1-3 (also called antibody 11-5) detecting an epitope in the N-terminal domain of MSBI 1 Rep (aa 1-136).

Anti-Rep antibodies of group D detect specifically a structural epitope of MSBI1 (SEQ ID NO:1), where antibody MSBI1 961-2-2 designated as "D1" (also called antibody 9-2 or AB9; DSM ACC3331) detects an epitope depicted in SEQ ID NO:9 (aa 281-287) in the C-terminal domain of MSBI1. Antibody MSBI1 761-5-1 (also called antibody 13; DSM ACC3328) designated as "D2" detects a 3D structural epitope of MSBI1 which is exclusively accessible under in vivo conditions and is not accessible in Western Blots. In immunofluoresence assays such anti-Rep antibodies detect specifically speckles (cytoplasmatic aggregations) of the Rep protein (often in the periphery of the nuclear membrane.

Based on the results of the examples, the inventors provided a schematic summary of the BMMF-induced indirect carcinogenesis in liver. Reference is made to Fig. 7. It shows in part (A) that early exposure to BMMF-containing diet leads to the enrichment of BMMF-positive macrophages contributing to chronic inflammation flanked by production of diffusing Reactive Oxygen Species (ROS)/Reactive Nitrogen Species (RNS), resulting in oxidative stress and induction of random DNA mutations in neighboring, replicating stem cells (and/or early daughter cells). Once several cancer driver mutations are fixed, dysplasia, and as a final consequence, cancer might develop after several decades. Fig. 7B shows that Rep expression was observed in the tumor-adjacent tissues of liver cancer and metastasis patients based on immunohistochemistry (IHC) and immunofluorescence (IF) analysis. The Rep expression was associated with CD68⁺ macrophages around the portal triad (PT) and increased in the liver of HCC and CRLM patients compared to healthy liver tissue, which may indicate a link between BMMFs and tumor-associated chronic inflammation in the liver. Exclusively in viral hepatitis-associated HCC, Rep expression was also detected in macrophages within the primary tumor; whether BMMFs play a pathophysiological role in chronic viral hepatitis-associated carcinogenesis may be contemplated. Specifically, in the liver of healthy individuals, a well-defined gradient-like Rep staining in hepatocytes (cytoplasmic speckled) was observed, which was strongest in hepatocytes around the central vein (CV) and decreased in intensity toward the PT. BMMF detection was supported by western Blot (WB), mass spectrometry and IEM (protein) and detection of BMMF DNA reads by *in silico* BMMF screening. Altogether, these observations indicate that the presence of BMMFs in the liver is associated with chronic inflammation and may represent a novel (synergistic) risk factor for liver cancer.

To test a possible association of BMMF with the induction of HCC and CRLM, the inventors carried out immunohistological detection and quantified BMMF Rep protein in liver tissues of HCC or CRLM patients, as well as in healthy liver tissues. Rep was expressed in all tumor-distant and peritumor tissue specimens of HCC and CRLM patients in hepatocytes and macrophages around PT region. Increased numbers of Rep⁺CD68⁺cells were found around the PT regions of tumor-distant and peritumor tissues of HCC and CRLM patients compared to healthy liver tissues. In addition, a case-vs.-control insensitive detection in hepatocytes was identified with strongest expression in tissue areas in close vicinity of hepatic ventral veins (CV).

As evidenced by the following examples, detection of BMMF Rep positive CD68⁺ macrophages around PT indicate a link between BMMFs and tumor-associated chronic inflammation in the liver. Therefore, the inventors assessed the correlation of the expression of BMMF Rep and CD68 as a marker for hepatic macrophages/Kupffer cells. An increased number of CD68⁺ macrophages was observed in the liver of HCC and CRLM patients compared to healthy livers. IF detection showed an increased number of Rep⁺CD68⁺ macrophages in the PT regions in tumor-distant and peritumor tissues of HCC (P=0.039 and P=0.025) and CRLM patients (P=0.054 and P=0.003) when compared to healthy controls. Rep⁺ macrophages with liver cancer-associated inflammation was supported by correlation analysis of Rep⁺ and CD68⁺ cells around PT. Importantly, it remains unknown whether these Rep⁺ macrophages in liver tissues of HCC and CRLM patients represent a distinct subpopulation of hepatic macrophages.

Rep⁺ macrophages in PT regions were detected in both DAB and IF detection using multiple, different anti-Rep antibodies. The effective blockade of detection in IHC with purified H1MSB.1 (also called MSBI1) Rep suggested the specificity of the staining. Rep antibody detection was also supported by WB on tissue lysates from liver cancer patients and revealing protein bands mainly at the size of 45 kDa (38 and 55 kDa bands occasionally) with 4 different Rep antibodies AB2, AB3, AB5 and AB10. IHC detection with Rep antibodies indicated no BMMF Rep detection in the tumor tissues of non-viral hepatitis-associated HCC and CRLM. However, WB showed detection of bands from lysates of tumor tissues, which might be partially explained by a larger fraction of tumor-adjacent/peritumor tissue content within the tissue specimen (tumor tissue heterogeneity). In addition, BMMF Rep protein detection was also supported by IEM on consecutive FFPE sections of IHC-positive liver tissues, where the inventors found similarly-shaped BMMF pleomorphic vesicular structures with high density gold-labeling. Importantly, the presence of BMMFs was also supported by identification of BMMF DNA reads in *in silico* BMMF screening analyses of human HCC patients. In addition to H1MSB.1 (also called MSBI1), several different BMMF isolates were identified in liver tumor cohorts e.g. C1HB.4 (also called HCBI4), H1MSB.2 (also called MSBI2) and C1MI.9M.1.

In addition, the inventors compared BMMF Rep expression in HCC patients with patients with chronic HBV vs. HCV infection, as several types of viruses might be able to replicate non-viral genetic elements such as BMMF plasmids. IHC and IF analysis of Rep expression revealed that Rep expression is also increased in peritumor viral-hepatitis linked HCC (comparable to non-viral hepatitis HCC peritumor tissues); but here, also the tumor tissues displayed strong Rep expression in cells close to the PT. Within tumor tissues, Rep positivity overlaps with CD68⁺ macrophages.

In conclusion, IHC analysis showed BMMF Rep protein detection in the liver of HCC and CRLM patients and in healthy livers of individuals (autopsy specimens). Immunofluorescence detection revealed an increased number of Rep⁺ CD68⁺ macrophages in tumor-distant and peritumor tissues of HCC and CRLM patients compared to healthy control liver around the PT of hepatic lobules, which may indicate chronic inflammation associated with BMMF. Rep was also expressed in hepatocytes with the highest detection levels observed in hepatocytes in close vicinity to the CV resulting in a staining gradient, which was most noticeable in healthy liver, but partly disrupted in patients. Only in patients with viral hepatitis-associated HCC the tumor tissue was positive for Rep as opposed to the tumor tissues of patients with non-viral hepatitis-associated HCC and CRLM, which indicates an interaction of BMMFs and hepatitis viruses in the context of liver cancer. The presence of BMMFs in liver could further be validated by multiple, independent methodologies such as western blotting, mass spectrometry analysis, IEM and in *in silico* analyses of HCC DNA and RNA sequencing datasets and BMMF DNA isolation from tissues of a CRLM patient.

The invention is further illustrated by, but not limited to, the following examples:

### EXAMPLES

### Example 1: General Material and Methods

### Ascertainment of patients and medical factors

All patients were treated by surgery at the National Centre for Tumor Diseases (NCT) Heidelberg and Department of Surgery of the University Hospital Mannheim with written informed consent given by all participants. The study was proofed by the ethics committee of Heidelberg (2021-293N-MA, biobanking vote and S-317/2023, study vote). Study parameters conformed to the standards of the Declaration of Helsinki. Medical data was extracted from a clinical documentation system. All diagnoses were reviewed by trained pathologists.

### Patient samples preparation

Tissue samples from formalin-fixed paraffin-embedded (FFPE) and fresh liver tissues from liver resection were provided by NCT tissue bank and University Hospital Mannheim in accordance with the local regulations. Resection specimens were fixed in 4% formaldehyde at room temperature over night to prepare FFPE tissue sections as described previously (26). FFPE tissue sections from patients with HCC (non-viral hepatitis-associated HCC N=9, viral hepatitis-associated HCC, HBV N=5 or HCV N=11), CRLM (N=14) and autopsy specimens of individuals with healthy liver tissue (Ct N=18) were used for histological analysis. Snap-frozen tissues and cryo-frozen tissue sections were stored under -80 °C until further processing for western-blotting (non-viral hepatitis-related HCC N=7, CRLM N=9). Tumor, peritumor and tumor-distant tissues from cancer patients were used for analysis. Peritumoral and tumor-distant tissue was defined as tissue within 0.5-1 cm or >1 cm in proximity to the tumor margin, respectively.

### Immunohistochemical and immunofluorescence detection

FFPE tissue sections were washed with 1xPBS, dehydrated with a standard ethanol series, followed by xylene and embedded in paraffin using standard procedures. FFPE tissue sections (4-6 µm thickness on superfrost slides, Zeiss, Oberkochen, Germany) were used for IHC and immunofluorescent (IF) detection using standard ethanol reagents, EDTA/citric acid for antigen retrieval at 98 °C 30 min, followed by primary antibody incubation at room temperature overnight and subsequent secondary antibody incubation at room temperature for 1 hour. Bright field DAB (Diaminobenzidine, Leica, Wetzlar, Germany) or fluorescence (Alexafluor dyes, Invitrogen, Waltham, Massachusetts) detection reagents were used as previously described (22,26). Different mouse monoclonal anti-Rep (targeting BMMF1 group H1MSBI.1 or consensus) (22) and anti-CD68 antibodies (rabbit, 1:500, Cell signaling, Danvers, Massachusetts) were used for the detection of BMMFs and macrophages, respectively. Different IgG isotype control antibodies were used for negative control staining. The specificity of the Rep antibodies was determined by incubating an antibody solution with or without purified Rep protein for 30 min RT on FFPE sections, followed by standard DAB detection (10-fold molar excess of purified H1MSB.1 Rep). Stained slides were scanned with the Hamamatsu NanoZoomer S60 (2.0-HT) slide scanner (Hamamatsu, Japan).

### Analysis of immunohistochemical and immunofluorescence detection

Semi-automated quantification of Rep and CD68 DAB staining on consecutive slides was conducted with the help of an in-house developed script for ImageJ/Fiji (V1.52)(27). The quantification was based on signal intensity of hematoxylin nuclear and antibody detection on digitalized scans after Rep and CD68 DAB expression of 6 randomly selected microscopic areas. Percentage (%) of positive area and positive cells (% with respect to total area or total cells) for Rep and CD68 were presented based on positive DAB signals. Quantification of digitalized scans of co-immunofluorescence staining for Rep⁺ and CD68⁺ macrophages or glutamine synthetase (GS⁺) cells was performed with a script based on DAPI nuclear and antibody detection with ImageJ/Fiji (v1.52) of 5-6 randomly selected microscopic areas as previously described (26). The data were presented as percentage of Rep⁺, CD68⁺ and Rep⁺CD68⁺ double positive cells or % GS⁺ cells (% with respect to total cells).

### Statistical analysis

Mann-Whitney U test and paired T -Test were used respectively for statistical analysis between different groups and paired tissues from the same individuals. Correlation analysis of Rep and CD68 staining in IF was determined by Pearson' rank correlation coefficient rho (GraphPad Prism8). P>0.05 shows no significant difference, P≤ 0.05 with significant difference, indicated by *P ≤ 0.05, ** P ≤ 0.01, *** P ≤ 0.001, **** P ≤ 0.0001.

### Western-blot (WB)

Snap frozen tissue pieces and cryo-frozen tissue sections (~30 mg) or FFPE tissue cuts (4x 10 µm, after deparaffinization) were incubated in urea lysis buffer (8 M urea, 100 mM NaH2PO4, 10 mM Tris pH 8.0 with protease inhibitors), followed by lysis in a tissue homogenizer (Bertin instruments, Montigny-le-Bretonneux, France, protocol: 3 x 20 s, hard, 4 °C)(22). After lysate clarification (centrifugation at 22,000x g, 4 °C,20 min) 25 µg protein per lane was loaded for SDS-PAGE (4-20% gels, Invitrogen, Waltham, Massachusetts), followed by transfer on nitrocellulose membranes (Turboblot, Bio-rad, Hercules, California) and immunodection as described previously (22) with anti-Rep antibodies AB 2, 3, 5 or 10.

### Mass spectrometry

Mass spectrometry analysis was conducted on the same protein lysates as used for WB in collaboration with the Proteomics Core Facility of the German Cancer Research Center. In brief, protein tissue lysates were applied to SDS-PAGE and gel pieces corresponding to four regions of interest (60-100 kDa, 45-60 kDa, 35-45 kDa and 20-30 kDa respectively) were excised, reduced, carbamidomethylated using iodoacetamide and tryptically digested for 4-hours before analyses on a machine Orbitrap Exploris 480 mass spectrometer (Thermo Fisher Scientific, Waltham, Massachusetts). Data analysis was conducted using MaxQuant (version 1.6.14.0). Quantification was done using a label-free quantification approach based on the MaxLFQ algorithm. The peptides were assigned using an up-to-date BMMF library containing possible peptides from all ORFs in all six reading frames.

### Example 2: BMMF Rep protein detection in the human liver

To investigate an association between BMMFs and liver cancer or metastasis, the presence of the conserved BMMF Rep protein using anti-Rep antibodies for IHC on FFPE tissue sections from HCC and CRLM patients and healthy liver samples (control, Ct; from autopsy specimens of individuals who died of non-infectious and/or non-cancerous diseases) was characterized. Tissue expressions were stratified in tumor, peritumor and tumor-distant areas of HCC and CRLM as well as healthy liver tissue specimens (Fig. 1A).

IHC with the Rep AB3 antibody on liver tissues from healthy controls (N=18), HCC (N=25) and CRLM (N=14) patients revealed antibody staining in all tissue specimens except tumoral tissue of patients with non-viral hepatitis-associated HCC or CRLM (Table 2). The strongest expression was observed in tumor-adjacent tissues (tumor-distant and peritumor, Fig. 1B).

**Table 2. Tissue positivity upon FFPE IHC-DAB staining with anti-Rep antibody AB3**

| | **Tissue types** | **Patients tested (N)** | **Patients positive (N)** | **Positivity (%)** |
|---|---|---|---|---|
| **Healthy liver (Ct)** | **Healthy** | **18** | **18** | **100** |
| **CRLM** | **Tumor-distant** | **14** | **14** | **100** |
| | **Peritumor** | **14** | **14** | **100** |
| | **Tumor** | **12** | **0** | **0** |
| **non-viral hepatitis-associated HCC** | **Tumor-distant** | **8** | **8** | **100** |
| | **Peritumor** | **8** | **8** | **100** |
| | **Tumor** | **9** | **0** | **0** |
| **viral hepatitis-associated HCC** | **Tumor-distant (cirrhosis)** | **9** | **9** | **100** |
| | **Peritumor** | **11** | **11** | **100** |
| | **Tumor** | **16** | **9** | **56%** |

### Example 3: BMMF Rep protein expression in hepatocytes and macrophages of non-viral hepatitis-associated HCC and CRLM patients through detection of BMMF Rep expression in HCC and CRLM upon immunohistochemistry

Next, the spatial distribution and subcellular levels of BMMF Rep protein expression were assessed. Immunohistochemical detection showed strong Rep positivity with AB3 in hepatocytes and macrophages of tumor-distant and peritumor tissues from non-viral hepatitis-related HCC and CRLM, as well as in healthy liver tissue specimens. No Rep staining was observed in the tumor tissue of non-viral hepatitis-related HCC and CRLM patients (Fig. 1B). Control detection with isotype control antibodies (IgG Ct) on consecutive sections showed no positivity, supporting specificity of the staining (Fig. 1B). Specificity of Rep AB3 detection was representative and supported based on additional individuals (Fig. 2A-B). The specificity of detection was further supported by pre-incubation of FFPE cuts with the purified H1MSB.1 Rep protein, showing effective blockade of antigen detection by anti-Rep AB3 antibody.

A trend towards increased (not significant) Rep-stained tissue area (% with respect to total area) was observed in tumor-distant (mean 6.1%) and peritumor (4.9%) of non-viral hepatitis-associated HCC compared to healthy liver tissue (4.1%, P=0.724 and P=0.534, respectively Fig.1C). Likewise, quantification on Rep-positive cells (% with respect to all cells) showed a (non-significant) trend of an increased number of Rep⁺ cells in tumor-distant (11.4%) and peritumor tissues (10.3%) of non-viral hepatitis-associated HCC compared to healthy liver tissue (8.5%, P=0.147 and P=0.209). Similarly, in CRLM patients, a trend for increased numbers of Rep⁺ cells in tumor-distant (10.4%) and peritumor tissues (10.9%) was observed compared to Ct (8.5%, P=0.123 and P=0.090). Concerning the area, a significantly increased area of Rep expression was observed for tumor-distant tissues (6.2%) and trend for peritumor tissues (6.4%) of CRLM patients compared with Ct (4.1%, P=0.033, P=0.054, Fig.1C). In general, Rep detection levels revealed large differences between individuals within each subgroup. Only in the tumoral tissue of HCC and CRLM patients, consistently low Rep detections were observed (Fig. 1C).

### Example 4: BMMF Rep protein detection by additional anti-Rep antibodies

On top of anti-Rep AB3, additional Rep antibodies have also been tested. Among 10 different anti-Rep antibodies (22) tested in IHC, AB3 and AB10 showed the strongest and most specific tissue expression patterns. Detection with anti-Rep AB10 showed similar expression intensities when compared to AB3 (Fig. 2C). AB1, AB5, AB11, AB14 and AB15 revealed positive detection with weaker intensity or smaller areas of the tissues (Fig. 2C).

The detection of Rep expression was corroborated by Western blotting (WB) using anti-Rep antibodies (Rep AB3, AB10, AB2 and AB5) and revealed bands mainly at around 45 kDa, occasionally at around 38 kDa and 55 kDa (Fig. 6). No bands were detected with other Rep antibodies.

### Example 5: Increased number of CD68⁺ macrophages in non-viral hepatitis-associated HCC and CRLM patients

Next immunohistochemical detection on consecutive cuts using an anti-CD68 antibody were conducted to test whether liver macrophages express Rep proteins (Fig. 1B). In non-viral hepatitis-associated HCC, quantification of CD68⁺ cells of DAB IHC staining revealed a higher number of CD68⁺ macrophages in tumor-distant (mean 23.9%) and peritumor tissue (27.2%) compared to Ct (15.7%, P=0.013 and P=0.001, Fig. 1D). This was also supported by increased CD68⁺ tissue area in tumor-distant (8.4%) and peritumor tissue (10.1%) compared to Ct (5.2%, P=0.025 and P<0.001, Fig. 1D). Similarly, in CRLM patients, increased numbers of CD68⁺ macrophages were identified in tumor-distant (22.4%) and peritumor tissue (25.0%) compared to Ct (15.7%, P=0.028 and P=0.001), as well as significantly increased CD68⁺ tissue area in tumor-distant (9.2%) and peritumor tissue (10.8%) compared to healthy liver (5.2%, P=0.021 and P<0.001, Fig. 1D).

### Example 6: Increased number of BMMF Rep⁺ macrophages around portal triads (PT) in non-viral hepatitis-associated HCC and CRLM patients

As the inventors observed a general association of Rep and CD68 by DAB IHC, they conducted co-immunofluorescence (IF) detection for Rep and CD68 (Fig. 3A). Importantly, co-staining of Rep and CD68⁺ macrophages was almost exclusively observed in the PT region. This is why Rep/CD68 quantifications were subsequently carried out focusing specifically on the PT region. In the PT region there was no statistical difference in the number of Rep⁺ cells (% with respect to all cells) in tumor-distant or peritumor tissues from patients with non-viral hepatitis-associated HCC and CRLM compared to Ct (all P>0.05, Fig. 3B-C). A trend towards an increase could be observed in peritumor of non-viral hepatitis-associated HCC (5.1%) and CRLM (5.2%) compared to Ct (4.1%, P=0.316 and P=0.245). Moreover, there was less or no detection of Rep in tumor tissue of patients with non-viral hepatitis-associated HCC and CRLM (Fig. 3B-C, left panel). Concerning CD68, an increased number of CD68⁺ macrophages was detected in tumor-distant (27.8%) and peritumor tissue (27.1%) of non-viral hepatitis-associated HCC compared to Ct (17.3%, P=0.003 and P=0.002, Fig. 3B). Similarly, an increased number of CD68⁺ macrophages was also observed in peritumor (24.2%) of CRLM vs. Ct (17.3%, P=0.009, Fig. 3C, middle panel).

Remarkably, an increased number of Rep⁺CD68⁺ macrophages was detected around the PT region in tumor-distant (1.5%) and peritumor tissues (1.7%) of patients with non-viral hepatitis-associated HCC compared to healthy liver Ct (0.9%, P=0.039 and P=0.025, Fig. 3B, right panel). Comparable data was observed for CRLM with increased numbers of Rep⁺CD68⁺ macrophages in tumor-distant (1.5%) and peritumor tissues (1.8%) vs. Ct (0.9%, P=0.054 and P=0.003, Fig. 3C, right panel). Correlation analysis of Rep and CD68 positive cells based on IF detection around the PT regions showed a significant correlation in tumor-distant (N=30) and peritumor (N=20) tissues of HCC and CRLM patients (Pearson r=0.58, P=0.0009; r=0.42, P=0.027).

### Example 7: BMMF Rep detection in tumor macrophages of viral hepatitis-associated HCC

To investigate whether BMMF expression is associated with chronic viral hepatitis, the inventors conducted IHC detection on FFPE sections of tumor-distant, peritumor and tumor tissues from HCC patients with chronic HBV (N=5) or HCV (N=11) infection. Firstly, no differences in the expression of Rep and CD68 were identified in tumor-distant and peritumor tissue of HBV- vs- HCV-infected HCC patients (Fig. 4A). However, in contrast to non-viral hepatitis-associated HCC, Rep expression was observed within the tumor tissue of viral hepatitis-associated HCC patients. IHC detection with Rep AB3 revealed a positive signal within the tumors of 9 out of 16 patients (56%) (Table 2).

A decreased number of Rep⁺ cells was found in tumor tissues (mean 2.8%) compared to tumor-distant (14.4%) and peritumor tissues (20.9%) in HBV-infected HCC patients (P=0.031; P=0.016, N=4, Fig. 4B). In HCV-infected HCC patients, no difference of Rep staining was observed between different tissue types. On the level of CD68⁺ macrophages (HCV), a significantly decreased number of CD68⁺ cells was identified in tumor (13.0%, N=10) vs. tumor-distant (28.2%) and peritumor tissues (20.0%) (P=0.001, N=5; P=0.030, N=6, Fig. 4B). Quantification of the positive tissue area did not reveal any significant difference between different regions of HCCs associated with either HBV or HCV infection or between HBV and HCV individuals.

Next, the inventors compared viral vs. non-viral hepatitis-associated HCC. Due to the low number of tested individuals per group and based on above statistical analysis in HBV and HCV-associated HCC, we combined the data from HBV and HCV-associated HCC individuals as viral hepatitis-associated HCC cohort. Comparison of the number of Rep and CD68 positive cells (Fig. 4C) as well as positive area showed no statistical difference in viral vs. non-viral hepatitis-associated HCC on the tumor-distant, peritumor and tumor tissues respectively (all P>0.05).

In addition, co-immunofluorescence (IF) detection of Rep and CD68⁺ macrophages on FFPE sections and quantification were carried out as described above. Rep expression in tumor tissues of viral hepatitis-associated HCC was predominantly found in macrophages (Fig.5A-B). Cell-based quantification by IF revealed an increased number of Rep⁺ cells in viral vs. non-viral hepatitis associated HCC in tumor-distant (8.1% vs. 3.6%, P=0.004) and tumor tissue (7.5% vs. 0.5%, P=0.0001, solid bar vs. dashed bar, Fig. 5C, left panel). Similarly, an increase of the number of CD68⁺ macrophages was identified in viral vs. non-viral hepatitis associated HCC in tumor-distant (44.9% vs. 27.8%, P=0.003) and tumor tissue (27.1% vs. 14.8%, P=0.033, Fig. 5C, middle panel). An increased number of Rep⁺CD68⁺ cells was detected only in tumor tissues of viral vs. non-viral hepatitis-associated HCC (2.7% vs. 0.2%, P=0.0005, N=15 or 8, Fig. 5C, right panel). There was almost no detection of Rep in the tumoral tissue of non-viral hepatitis-associated HCC as consistent with IHC staining.

### Example 8: Disrupted hepatic gradient of BMMF expression around CV in HCC and CRLM

Rep expression was not exclusively observed in PT macrophages, but also in cytoplasmic speckles within hepatocytes. When analysing hepatocyte staining in more detail, AB3 detection of hepatocytes around the central vein (CV) showed highest expression levels which decreases gradually in hepatocytes localized towards PT in healthy individuals. In contrast, in non-viral hepatitis-associated HCC, the Rep expression was more homogeneously distributed throughout the peritumoral tissue resulting in a less-clearly identifiable zonation gradient.

### SEQUENCE SUMMARY

| SEQ ID NO | SEQUENCE |
|---|---|
| 1 | Amino acid sequence of Rep protein encoded by MSBI1.176 |
| | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVF |
| 2 | Amino acid sequence of Rep peptide fragment |
| | EARETGKGINANDPLTVH |
| 3 | Amino acid sequence of Rep peptide fragment |
| | KQINEHTDITASYEQHKKGRT |
| 4 | His-Tag (with two neutral stuffer amino acids) |
| | GAHHHHHH |
| 5 | T7-Tag |
| | MASMTGGQQMG |
| 6 | FLAG-Tag |
| | DYKDDDDK |
| 7 | Strep-II-Tag |
| | WSHPQFEK |
| 8 | Amino acid sequence of Rep protein encoded by MSBI2.176 |
| | MSKLVVKDNALMNASYNLDLVEQRLILLAIIEARESGKGINANDPLTVHA ESYINQFGVHRVTAYQALKDACDNLFARQFSYQSKSEKGNIQNHRSRWVS EIIYIDTEATVKIIFAPAIVPLITRLEEQFTKYDIEQISDLSSAYAIRLY ELLICWRSTGKTPIIGLGEFRNRVGVLDSEYHRIAHLKERVIEHSIKQIN EHTDITATYEQHKKGRTITGFSFKFKQKKPKQAEIATETPKTATNDPDTT KPLTEPQIAKYSMILCKLGSISDLSNFPDYPAFANWIGNILRNPEKADEQ IAKRIFTALKTETDYSKKN |
| 9 | MSBI.1 specific epitope |
| | NRLSDRF |
| 10 | Amino acid sequence of Rep protein encoded by CMI1.252 |
| | MSDLIVKDNALMNASYNLALVEQRLILLAILEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISELSSAYAVRLYELLICWRSTGKTPIIDLTEFRKRLG VLDTEYTRTDNLKMRVIELGLKQINEHTDITASYEQHKKGRTITGFSFKFKQKKKTGA EMPKNSDSSPHIEKPSQIPANIAKQPENAKKDDLGHRASKITGLIMSNGLADRFKRGD ESVIDMMKRIKEEITTDTTADQWENKLEEFGVIFQS |
| 11 | Amino acid sequence of Rep protein encoded by CMI2.214 |
| | MSDLIVKDNALMNASYNLDLVEQRLILLAILEARETGKGINANDPLTVHAESYINQFG VARQTAYQALKDACKDLFARQFSYQEKRERGRANITSRWVSQIAYIDETATVEVIFAP AVVPLITRLEEQFTQYDIEQISGLSSAYAVRLYELLICWRSTGKTPVIELAEFRKRLG VLNDEYTRSDNFKKWIIENPIKQINEHTDITASYEQHKKGRTITGFSFKFKQKKKTEP ETPKNSDSSQRIEKPSQIPANIVKQPENANLSDLQHRASKITGLIMSNRLSDRFKQGD ESIMQMMARIQSEITTDSIADQWQSKLEEFGVVF |
| 12 | Amino acid sequence of Rep protein encoded by CMI3.168 |
| | MSDLIVKDNALMNASYNLALVEQRLILLAILEARETGKGINANDPLTVHASSYINQFN VERHTAYQALKDACKDLFARQFSYQEKRERGRANITSRWVSQIAYIDETATVEVIFAP AVVPLITRLEEQFTQYDIEQISGLSSAYAVRLYELLICWRTTGKTPVLDLTEFRKRLG VLDTEYTRTDNLKMRVIEQSLKQINKHTDITASYEQHKKGRTITGFSFKFKQKKKTEP ETPKNNDSGVSKPKTVEIPAEVVKQPKNTNLSDLEKRVRMITGAIAKNNLASRFQHGN ESPLDMMKRIQSEITSDETADLWQNKLESMGVVF |
| 13 | DNA sequence MSBI1 Rep codon-optimized |
| | ATGAGCGACCTGATCGTGAAAGACAATGCCCTGATGAACGCCTCCTACAACCTGGCAC TGGTCGAACAGAGACTGATTCTGCTGGCTATCATCGAGGCAAGGGAGACCGGCAAGGG CATCAACGCCAATGACCCCCTGACAGTGCACGCCAGCTCCTACATCAACCAGTTTAAT GTGGAGCGCCACACCGCCTATCAGGCCCTGAAGGACGCCTGCAAGGATCTGTTTGCCC GGCAGTTCAGCTACCAGGAGAAGCGGAGAGAGGCAGGATCAACATCACAAGCAGATG GGTGTCCCAGATCGGCTATATGGACGATACCGCCACAGTGGAGATCATCTTTGCACCA GCAGTGGTGCCTCTGATCACCAGGCTGGAGGAGCAGTTCACACAGTACGACATCGAGC AGATCTCCGGACTGTCTAGCGCCTACGCCGTGCGCATGTATGAGCTGCTGATCTGTTG GCGGTCTACCGGCAAGACACCTATCATCGAGCTGGATGAGTTCCGCAAGCGGATCGGC GTGCTGGACACCGAGTACACCAGAACAGATAACCTGAAGATGAGAGTGATCGAGCTGG CCCTGAAGCAGATCAATGAGCACACCGATATCACAGCCTCTTATGAGCAGCACAAGAA GGGCCGCGTGATCACCGGCTTCAGCTTTAAGTTCAAGCACAAGAAGCAGAACTCTGAC AAGACACCAAAGAATAGCGATTCCTCTCCCCGGATCGTGAAGCACAGCCAGATCCCTA CCAACATCGTGAAGCAGCCAGAGAATGCCAAGATGTCCGACCTGGAGCACAGGGCATC TAGGGTGACAGGCGAGATCATGAGAAATAGGCTGAGCGATCGGTTCAAGCAGGGCGAC GAGTCCGCCATCGATATGATGAAGAGAATCCAGTCCGAGATCATCACCGACGCCATCG CCGATCAGTGGGAATCTAAACTGGAAGAGTTTGGAGTCGTGTTTGGAGCACATCACCA TCATCATCACTGA |
| 14 | Protein sequence MSBI1 Rep codon-optimized |
| | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVFGA |
| 15 | DNA sequence MSBI1 Rep wild-type |
| | ATGAGCGATTTAATAGTAAAAGATAACGCCCTAATGAATGCTAGTTATAACTTAGCTT TGGTTGAACAGAGGTTAATTCTATTAGCAATCATAGAAGCGAGAGAAACAGGCAAAGG GATTAATGCCAATGATCCTTTAACAGTTCATGCAAGTAGCTATATCAATCAATTTAAC GTAGAAAGGCATACGGCATATCAAGCCCTCAAAGATGCTTGTAAAGACTTGTTTGCCC GTCAATTCAGTTACCAAGAAAAGCGAGAACGAGGACGAATTAATATTACAAGTCGATG GGTTTCGCAAATTGGCTATATGGACGATACAGCAACCGTTGAGATTATTTTTGCCCCT GCGGTTGTTCCTCTGATTACACGGCTAGAGGAACAGTTCACCCAGTACGATATTGAGC AAATTAGCGGTTTATCGAGTGCATATGCTGTTCGTATGTACGAACTGCTGATTTGTTG GCGTAGCACAGGCAAAACACCAATTATTGAGCTAGACGAGTTTAGAAAGCGAATAGGT GTTTTAGATACTGAATACACTAGAACAGATAATTTAAAGATGCGAGTTATTGAATTAG CCCTAAAACAAATCAACGAACATACAGACATCACAGCAAGCTATGAACAACACAAAAA AGGGCGAGTGATTACAGGATTCTCATTCAAGTTTAAGCACAAGAAACAAAACAGCGAT AAAACGCCAAAAAATAGCGATTCTAGCCCACGTATCGTAAAACATAGTCAAATCCCTC CAACATTGTAAAACAGCCTGAAAACGCCAAAATGAGCGATTTAGAACATAGAGCGAGC CGTGTTACAGGGGAAATAATGCGAAATCGTCTGTCAGATCGGTTTAAACAAGGCGATG AATCAGCAATCGACATGATGAAACGTATTCAAAGTGAAATAATAACCGATGCAATAGC AGACCAGTGGGAAAGCAAACTGGAGGAGTTTGGCGTGGTTTTTTAG |
| 16 | Amino acid sequence of Rep protein encoded by CMI4.158 |
| | MSDLIVKDNALMNASYNLALVEQRLILLAILEARETGKGINAND PLTVHANSYINQFNVERHTAYQALKDACKDLFARQFSYQEKRERGRINITSRWVSQIG YMDDTATVEVIFAPAVVPLITRLEEQFTQYDIEQISGLSSAYAVRLYELLICWRSTGK TPVIELAEFRKRIGVLDTEYTRTDNLKMRVIELALKQINEHTDITASYEQHKKGRTIT GFSFKFKQKKKTELETPKNSDSSPHIEKPSQIPTNIVKQPENAKKDDLGHRASKITGL IMSNGLADRFKRGDESVMNMMKRIKEEITTDATADLWQNKLESMVWFFSHD |
| 17 | Amino acid sequence of Rep protein encoded by HCBI3 |
| | MYRWFMSDLIVKDNALMNASYNLDLVEQRLVLLAILEARESGKGINANDP LTVHAESYINQFGVARQTAYQALKGACKDLFARQFSYQEKRERGRANITS RWVSQIAYIDETATVEVIFAPAVVPLITRLEEQFSKYDIEQISSLSSAYA VRLYELLICWRTTGKTPVIDLTEFRKRIGVLDTEYQRMDVFKRGVLELAL KQVNEHTDITATYEQHKKGRVITGFSFKFKQKKPKQAEIATETPKTATTD LATTKPLTEPQIAKYSMILCKLGSISDLGNFPDYPTFANWIANILRTPEK ADEQIAKRIFTALKTETDYSKKN |
| 18 | Amino acid sequence of Rep protein encoded by HCBI4 |
| | MKNGLVVKDNALMNASYNLEVTEQRLILLAIISARETGQGITSD SKLEIHASDYAIQFSVAKETAYEALKSAVNNLFERQFSFREETKKGTGIVRSRWVSRI KYIDDAAILEITFAPDVVPLITRLEEHFTSYQIKQIAQLTSKYAIRLYELLIAWRTTG KVPELELSEFRNRLGIASNEYTAMNNFKSRVLEPSIKQINEHTDITVTYEQHKKGRTI TGFSFRFKQKQQAKKIETNRDPNTPDFFIKMTDAQRHLFANKMSEMPEMGKYSQGTES YQQFAIRIADMLLQPEKFRELYPILEKAGFKE |
| 19 | Amino acid sequence of Rep protein encoded by C1MI.3M.1 |
| | MSELIVKDNALMNASYNLDLVEQRLILLAILEARESGKGINANDPLTVHA ESYINQFGVHRTTAYQALKDACKDLFARQFSYQEKRERGRANITSRWVSQ IAYIDETGTVEVIFAPAVVPLITRLEEQFTKYDIEQISGLSSAYAVRLYE LLICWRTTGKTPIIDLTEFRKRIGVLDTEYQRMDVFKRGVLELALKQVNE HTDITASYEQHKKGRVITGFSFKFKQKKPKQAEIATETPKTATNDPDTTK PLTEPQIAKYSMILCKLGSISDLSNFPDYPAFANWIGNILRNPEKADEQI AKRIFTALKTETDYSKKN |
| 20 | Amino acid sequence of Rep protein encoded by C1MI.9M.1: |
| | MRLMSELVVKDNALMNASYNLDLVEQRLILLAIVEARESGKGIN ANDPLTVHAESYINQFGVARQTAYQALKDACDDLFARQFSYQEINERGNTENVRSRWV SEVRYVDAEATVKLIFAPIVIPLITRLEERFTQYEMKQISELSTGYAIRLYELLICWR ATGKTPVIELADFRQRMGVLDSEYQRMYDLKKYVLEPSLKQINEHTDITATYEQHKKG RVITGFSFKFKQKKPKQAEIATETPKTATNDQNTIKPLTEPQIAKYSMILCKLGSISD LSNFPDYPSFANWIANILRTPEKADEQAAKRIFTALKTETDYSKKN |

### REFERENCES:

1. Funk, M., et al. (2014). "Isolation of protein-associated circular DNA from healthy cattle serum". Genome Announc 2(4)
2. Gunst, K., et al. (2014). "Isolation of bacterial plasmid-related replication-associated cirular DNA from a serum sample of a multiple sclerosis patient." Genome Announc 2(4).
3. Whitley, C., et al. (2014). "Novel replication-competent cirulara DNA molecules from healthy cattle serum and milk and multiple sclerosis-affected human brain tissue." Genome Announc 2(4).
4. zur Hausen, H., Bund, T., de Villiers, E.-M. (2017). "Infectious agents in bovine red meat and milk and their potential role in cancer and other chronic diseases." Curr. Top.Microbiol. Immunol., Volume 407, 83-116.
5. Slot, J. W. and H. J. Geuze (2007). Cryosectioning and immunolabeling. Nat Protoc 2(10): 2480-2491.
6. Li X, Ramadori P, Pfister D, Seehawer M, Zender L, Heikenwalder M. The immunological and metabolic landscape in primary and metastatic liver cancer. Nat Rev Cancer 2021;21:541-57
7. Falida, K., S. Eilebrecht, K. Gunst, H. zur Hausen and E. M. de Villiers (2017). Isolation of Two Virus-Like Circular DNAs from Commercially Available Milk Samples. Genome Announc 5(17):e00266-17. doi: 10.1128/genomeA.00266-17.
8. de Villiers, E. M. and H. Zur Hausen (2021). "Bovine Meat and Milk Factors (BMMFs): Their Proposed Role in Common Human Cancers and Type 2 Diabetes Mellitus." Cancers (Basel) 13(21):5407. doi: 10.3390/cancers13215407.
10. zur Hausen H, de Villiers EM. Dairy cattle serum and milk factors contributing to the risk of colon and breast cancers. Int J Cancer 2015;137:959-67
11. zur Hausen H, Bund T, de Villiers EM. Infectious Agents in Bovine Red Meat and Milk and Their Potential Role in Cancer and Other Chronic Diseases. Viruses, Genes, and Cancer 2017;407:83-116
12. Zelber-Sagi S, Noureddin M, Shibolet O. Lifestyle and Hepatocellular Carcinoma What Is the Evidence and Prevention Recommendations. Cancers 2022;14
13. Ma Y, Yang W, Li T, Liu Y, Simon TG, Sui J, et al. Meat intake and risk of hepatocellular carcinoma in two large US prospective cohorts of women and men. Int J Epidemiol 2019;48:1863-71
14. Manuelidis L. Nuclease resistant circular DNAs copurify with infectivity in scrapie and CJD. Journal of Neurovirology 2011;17:131-45
15. de Villiers EM, Gunst K, Chakraborty D, Ernst C, Bund T, zur Hausen H. A specific class of infectious agents isolated from bovine serum and dairy products and peritumoral colon cancer tissue. Emerg Microbes Infect 2019;8:1205-18
16. Konig MT, Fux R, Link E, Sutter G, Martlbauer E, Didier A. Circular Rep-Encoding Single-Stranded DNA Sequences in Milk from Water Buffaloes (Bubalus arnee f. bubalis). Viruses 2021;13
17. Konig MT, Fux R, Link E, Sutter G, Martlbauer E, Didier A. Identification and Characterization of Circular Single-Stranded DNA Genomes in Sheep and Goat Milk. Viruses 2021;13
18. Konig MT, Frolich K, Jandowsky A, Knauf-Witzens T, Langner C, Dietrich R, et al. First Insights into the Occurrence of Circular Single-Stranded DNA Genomes in Asian and African Cattle. Animals (Basel) 2023;13
19. Habermann D, Klempt M, Franz CMAP. Identification and Characterization of Novel SPHINX/BMMF-like DNA Sequences Isolated from Non-Bovine Foods. Genes 2023;14
20. Mobaraki G, Shi S, Smits KM, Severens K, Lommen K, Rennspiess D, et al. Bovine Meat and Milk Factor-like Sequences Are Frequently Detected in Renal Cell Carcinoma Tissues. Cancers (Basel) 2024;16
21. Eilebrecht S, Hotz-Wagenblatt A, Sarachaga V, Burk A, Falida K, Chakraborty D, et al. Expression and replication of virus-like circular DNA in human cells. Scientific Reports 2018;8
22. Bund T, Nikitina E, Chakraborty D, Ernst C, Gunst K, Boneva B, et al. Analysis of chronic inflammatory lesions of the colon for BMMF Rep antigen expression and CD68 macrophage interactions. Proceedings of the National Academy of Sciences of the United States of America 2021;118
23. Nikitina E, Burk-Körner A, Wiesenfarth M, Alwers E, Heide D, Tessmer C, et al. Bovine meat and milk factor protein expression in tumor-free mucosa of colorectal cancer patients coincides with macrophages and might interfere with patient survival. Molecular Oncology 2023;18(5):1076-1092.
24. zur Hausen H, Bund T, de Villiers EM. Specific nutritional infections early in life as risk factors for human colon and breast cancers several decades later. Int J Cancer 2019;144:1574-83
25. de Villiers EM, zur Hausen H. Bovine Meat and Milk Factors (BMMFs): Their Proposed Role in Common Human Cancers and Type 2 Diabetes Mellitus. Cancers 2021;13
26. Nikitina E, Alikhanyan K, Nessling M, Richter K, Kaden S, Ernst C, et al. Structural expression of bovine milk and meat factors in tissues of colorectal, lung and pancreatic cancer patients. Int J Cancer 2022;153(1):173-182.
27. Schindelin J, Arganda-Carreras I, Frise E, Kaynig V, Longair M, Pietzsch T, et al. Fiji: an open-source platform for biological-image analysis. Nat Methods 2012;9:676-82
28. Consortium TITP-CAoWG. Pan-cancer analysis of whole genomes. Nature 2020;578:82-93
29. Hutter C, Zenklusen JC. The Cancer Genome Atlas: Creating Lasting Value beyond Its Data. Cell 2018;173:283-5
30. Horak P, Uhrig S, Witzel M, Gil-Farina I, Hutter B, Rath T, et al. Comprehensive genomic characterization of gene therapy-induced T-cell acute lymphoblastic leukemia. Leukemia 2020;34:2785-9
31. Hadi R, Shin K, Reder N, Alpert L, Koch L, Choi WT, et al. Utility of glutamine synthetase immunohistochemistry in identifying features of regressed cirrhosis. Mod Pathol 2020;33:448-55
32. Krenkel O, Tacke F. Liver macrophages in tissue homeostasis and disease. Nat Rev Immunol 2017;17:306-21
33. Kietzmann T. Liver Zonation in Health and Disease: Hypoxia and Hypoxia-Inducible Transcription Factors as Concert Masters. International Journal of Molecular Sciences 2019;20
34. McEnerney L, Duncan K, Bang BR, Elmasry S, Li M, Miki T, et al. Dual modulation of human hepatic zonation via canonical and non-canonical Wnt pathways. Experimental and Molecular Medicine 2017;49
35. Panday R, Monckton CP, Khetani SR. The Role of Liver Zonation in Physiology, Regeneration, and Disease. Semin Liver Dis 2022;42:1-16

## Claims

1. Use of Bovine Meat and Milk Factor Group 1 (BMMF1) Rep Protein together with CD68 as a diagnostic marker for liver cancer.

2. The use of claim 1 wherein the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep), CMI3 genome-encoded Rep protein (CMI3 Rep), CMI4 genome-encoded Rep protein (CMI4 Rep), HCBI6 genome-encoded Rep protein (HCBI6 Rep), HCBI4 genome-encoded Rep protein (HCBI4 Rep), C1 MI3M.1 genome-encoded Rep protein, C1MI.9M.1 genome-encoded protein (C1MI.9M.1 Rep).

3. A method for diagnosing, or determining a predisposition for, liver cancer or liver metastasis in a human subject, comprising:
- detecting whether a Bovine Meat and Milk Factor Group 1 (BMMF1) DNA-replication associated protein (Rep protein) is present in a sample isolated from a subject, comprising
- contacting the sample with an anti-BMMF1 Rep antibody and an anti-CD68 antibody;
- detecting binding between BMMF 1 Rep protein and the anti-BMMF1 Rep antibody, wherein detecting the binding between BMMF1 Rep protein and the anti-BMMF1 Rep antibody indicates that BMMF1 Rep is present in the sample,
- detecting binding between CD68-positive cells and the anti-CD68 antibody, wherein detecting the binding between the CD68-positive cells and the anti-CD68 antibody indicates that CD68-positive cells are present in the sample, and
- providing the diagnosis of liver cancer/metastasis or the predisposition for liver cancer to the subject when the presence of both BMMF1 Rep protein and of CD68 positive cells in the sample is detected.

4. The method of claim 3, wherein the antibody specific for Rep protein binds to an epitope that is within an amino acid sequence selected from the group consisting of amino acids from 1 to 136, from 137 to 229 and from 230 to 324 of SEQ ID NO:1.

5. The method of claim 3 or 4, wherein the sample from a subject is selected from the group consisting of a cancerous liver tissue, peripheral tissue surrounding the cancerous tissue, pre-cancerous dysplastic lesions and pre-cancerous cirrhotic lesions.

6. The method of any of claims 3 to 5, wherein additionally CD163 positive cells are detected in the sample by an anti-CD163 antibody.
